# EUROPEAN PATENT APPLICATION

(11) **EP 4 706 660 A1**
(43) Date of publication of application: **11.03.2026**
(21) Application number: 24800137.2
(22) Date of filing: 31.01.2024
(51) Int. Cl.: A61K 31/58, A61K 31/42, A61K 31/352, A61K 36/23, A61K 31/191, A61P 17/14, A61K 9/127, A61K 9/51

(54) **PHARMACEUTICAL COMPOSITION COMPRISING TELOMERASE ACTIVATOR AND NANOPARTICLES FOR DRUG DELIVERY, AND COMPOSITION CONTAINING SAME FOR PREVENTION, ALLEVIATION, OR TREATMENT OF HAIR LOSS**

(30) Priority: 04.05.2023 KR 20230058099
(71) Applicant: Aribio Co., Ltd., Seongnam-si, Gyeonggi-do 13535 (KR); Biochemgen Inc., Cheonan-si Chungcheongnam-do 31116 (KR)
(72) Inventor: CHOUNG, Jaijun, Seoul 06194 (KR); SEO, Boseung, Gunpo-si Gyeonggi-do 15836 (KR); SHIN, Deug Y, Yongin-si Gyeonggi-do 17098 (KR)
(74) Representative: Vossius & Partner Patentanwälte Rechtsanwälte mbB
(86) International application number: PCT/KR2024/001466
(87) International publication number: WO 2024/228448

(57) **Abstract**

The present invention relates to a pharmaceutical composition comprising a telomerase activator and nanoparticles for drug delivery and a composition containing same for the prevention, alleviation, or treatment of hair loss. More specifically, the present invention relates to a composition effective for promoting the regeneration and growth of hair follicles, the composition being a nanoliposome, nano-liposome, nano-silica particle, or nano-bubble composition in which drugs such as the plant extract component TA-65 and a derivative thereof that increase telomerase activity, and the telomerase activating compound GPC and a derivative thereof are encapsulated

## Description

### Technical Field

The present invention relates to a pharmaceutical composition for drug delivery comprising a telomerase activator and nanoparticles, and a composition containing the same for the prevention, improvement, or treatment of hair loss. More specifically, the invention provides a composition effective for promoting the regeneration and growth of hair follicles, comprising nanoliposomes, nano-silica particles, or nanobubbles in which drugs such as TA-65, a plant extract component that increases telomerase activity, and its derivatives, as well as GPC, a telomerase-activating compound, and its derivatives, are encapsulated.

### Background Art

Hair loss generally refers to the loss of thick, black hair on the scalp. Although the causes of hair loss are diverse, genetic factors and the male hormone androgen are considered major contributors. Among them, androgenic alopecia, which accounts for approximately 60-70% of all cases, occurs when testosterone is converted into dihydrotestosterone (DHT) by the enzyme 5-alpha reductase (SRD5A). The excessive DHT then binds to the androgenic receptor (AR) of dermal papilla cells (DPCs), inducing apoptosis and causing the miniaturization of hair follicles, which leads to progressive hair loss. Among the isozymes of 5-alpha reductase, type 2 (SRD5A2), which is mainly distributed in the dermal papilla and the outer root sheath of the hair follicle, plays a key role. Individuals with high expression levels or elevated enzymatic activity of SRD5A2 produce a relatively greater amount of DHT than average males, resulting in a higher likelihood of developing hair loss. Therefore, the key therapeutic strategy for androgenic alopecia lies in reducing the amount or activity of type 2 5-alpha reductase to prevent the conversion of testosterone into DHT.

Telomeres are repetitive DNA sequences (TTAGGG) located at the ends of chromosomes, known to stabilize the structure and function of chromosomes. The length of telomeres shortens as cellular DNA replication repeats. When the telomere length decreases below a critical threshold, the cell enters a senescent state where it can no longer proliferate. Thus, telomere length serves as a biomarker representing the degree of cellular aging. Telomere shortening occurs naturally with aging and can also be accelerated by factors such as stress, smoking, pollution, lack of exercise, and obesity. Moreover, the incidence of various age-related diseases, including cardiovascular disorders, increases as telomeres shorten. Cellular aging can therefore be considered a driving factor behind the aging of tissues and organs in the human body.

Telomerase is an enzyme complex that catalyzes the addition of telomeric repeat sequences to the 3' end of telomeres, thereby restoring telomere length shortened by DNA replication and various external stresses. Since the discovery of telomerase, much scientific attention has focused on its role in cancer, as telomerase is necessary for the continuous proliferation of cancer cells, leading to the assumption that telomerase expression might induce carcinogenesis. However, recent studies using genetically modified mice have demonstrated that high-level expression of telomerase does not induce cancer but rather restores physiological functions impaired by aging, suggesting its role in regeneration and rejuvenation.

Gene-knockout mice lacking the telomerase gene exhibit infertility, osteoporosis, diabetes, and neurological impairment, showing accelerated aging compared to normal mice. Conversely, in genetically engineered mice designed to express telomerase only upon oral administration of doxycycline, the induction of telomerase expression after aging resulted in the recovery of functions in organs such as the brain, reproductive system, spleen, liver, and intestine. Furthermore, telomerase activation was also found to promote hair follicle regeneration and reinitiate hair growth. These findings demonstrate that activation of telomerase induces the regeneration of aged hair follicles by stimulating the proliferation and renewal of hair follicle stem cells.

The discovery that telomerase activation can reverse various aging phenomena in gene-engineered animal studies has accelerated the search for telomerase-activating substances suitable for human application, leading to the identification of various telomerase activators.

Telomerase activators can be classified into two categories depending on their source:
(1) plant-derived extracts and their derivatives exhibiting telomerase-activating effects, and
(2) synthetic chemical compounds.

Cycloastragenol, a plant constituent derived from *Astragalus* species, and its derivatives are representative telomerase activators. Cycloastragenol (CAG, chemical formula C ₃₀H₅₀O₅) is also known as TAT2 or GRN665. TA-65, developed by TA Sciences (USA), is a telomerase activator extracted and purified from *Astragalus* plants using a patented method, and it contains cycloastragenol as an active ingredient. Other plant-derived telomerase activators include genistein (from soy), extracts of *Centella asiatica,* and maslinic acid derived from olive-pomace oil.

Among the chemically synthesized telomerase activators, GRN510 (Le Saux et al., 2013) and C3-(L)-valyl-cycloastragenol (US 9,913,852 B2) have been reported as cycloastragenol derivatives.

Additionally, N-propyl-5-(2-thienyl)isoxazole-3-carboxamide (US 2009/0143451 A1) and its various derivatives have been reported to enhance telomerase expression. One of these derivatives, guanidinopentyl phenylisoxazolecarboxamide (GPC; chemical formula C₁₆H₂₁N₅O₂), is known as a representative telomerase-activating compound.

Other compounds such as AGS499 and AGS500, reported by Shi et al. (2020), have also demonstrated telomerase-activating activity.

Although several studies and patents have reported the recovery of organ functions such as brain, reproductive organs, spleen, liver, and intestine, as well as the enhancement of immune activity using the aforementioned telomerase activators, no prior research or patent has reported delivering these telomerase-activating substances into hair follicles to activate follicular cells and improve hair loss or promote hair growth. Accordingly, the present inventors have conducted research and development to determine whether telomerase-activating substances exhibit efficacy in preventing or improving hair loss.

### DETAILED DESCRIPTION OF INVENTION

### Technical Problem

The object of the present invention is to provide a drug delivery composition using nanobubbles, nanoliposomes, nanoparticles, etc., or a method for preparing the same, and a composition for preventing, improving, or treating hair loss containing the same as an active ingredient, by inducing the regrowth of aged hair follicles that have entered a complete telogen due to aging and have ceased hair growth, thereby resuming hair growth through the use of a telomerase activator.

The present invention provides nanoparticles encapsulated with a telomerase activator as a hair loss treatment drug, and a composition containing the same for improving or treating hair loss.

More specifically, the object of the present invention is to provide a composition effective for promoting the regeneration and growth of hair follicles, the composition being a nanoliposome, nanosilica particle, or nanobubble in which drugs such as the plant extract component TA-65 and its derivatives, and the telomerase-activating compound GPC and its derivatives, which increase telomerase activity, are encapsulated.

### Technical Solution

The present invention relates to a conjugated structure of nanoliposomes, mesoporous silica nanoparticles, or nanobubbles encapsulating a telomerase activator.

The telomerase-activating substance is a substance that increases cellular telomerase expression or telomerase enzymatic activity, and is selected from one or more of the plant-derived extract component TA-65 or the compound GPC, and the present invention relates to a composition for improving or treating hair loss comprising the same.

In the present invention, TA-65 and GPC may each be formulated in nanobubble, nanoliposome, or nanosilica solutions at concentrations ranging from 0.2 mg/L to 2 g/L.

In addition, the hair follicle regeneration-promoting agent may be a substance capable of increasing the expression or enzymatic activity of telomerase.

The nanoliposomes may comprise lecithin, cholesterol, and cationic, anionic, or zwitterionic phospholipids.

The nanobubbles are preferably gas bubbles having a size of 50 nm to 500 nm, generated by various methods such as electrical, ultrasonic, or pressure techniques, so that they may penetrate into hair follicles, although larger sizes may also be included.

The silica nanoparticles are mesoporous silica nanospheres (MSN) having empty pores with sizes of 2 nm to 50 nm, and may be selected from M41S series silica nanoparticles with uniform pore size and volume, such as hexagonal MCM-41 (Mobile Crystalline Material-41). The composition and structure of the silica nanoparticles may vary. In the present invention, 100 nm MCM-41 nanoparticles purchased from Sigma-Aldrich, USA, were used as the silica nanoparticles.

The present invention may provide a composition for improving or treating hair loss, comprising nanoliposomes, nanobubbles, or mesoporous silica nanoparticles encapsulating a telomerase-activating substance.

### EFFECT OF INVENTION

The present invention relates to a composition for improving or treating hair loss, comprising nanoliposomes or mesoporous silica nanoparticles encapsulating a telomerase-activating substance, such as a plant extract component like TA-65 or a compound like GPC, or nanobubbles combined with such substances.

When a solution of the telomerase-activating substance dissolved in standard lotion solution was applied to aged animals, no hair growth-promoting or hair loss-improving effects were observed. This result explains why previous studies and patents have not reported the hair loss-improving effects of telomerase-activating substances.

However, activation of telomerase induces the regeneration of aged hair follicles and the activation of hair follicle stem cells. This has been demonstrated in experiments using genetically modified mice. Specifically, when the telomerase gene is expressed in aged mice whose hair follicles have entered a dormant state and hair growth has ceased, the regrowth of hair follicle stem cells, the regeneration of hair follicles, and the resumption of hair growth are observed.

The lack of reports that telomerase-activating substances induce hair follicle stem cell regrowth and hair growth can be attributed to the inability of these substances to penetrate into hair follicles and activate telomerase when merely applied topically to the scalp. Therefore, they fail to induce hair follicle regeneration and promote hair growth.

Accordingly, it is important to enhance the delivery efficiency of telomerase-activating substances into the interior of hair follicles using drug delivery carriers. The use of nanobubbles, nanoliposomes, and mesoporous silica nanoparticles in the present invention demonstrates that telomerase-activating substances exhibit efficacy as hair loss-improving and therapeutic agents. This approach can effectively treat hair loss caused by the aging of hair follicles and the cessation of hair growth.

Although nanoliposomes, mesoporous silica nanoparticles, and nanobubbles have been noted as drug delivery vehicles, they have not been previously used to deliver telomerase-activating substances specifically into aged hair follicles.

In the present invention, nanobubbles, nanoliposomes, and mesoporous silica nanoparticles containing telomerase-activating substances each exhibited a certain degree of hair growth and hair follicle regeneration efficacy. Therefore, while the extent of efficacy may vary depending on the composition of the drug and the delivery vehicle, these compositions demonstrate effectiveness as agents for improving and treating hair loss.

### BRIEF DESCRIPTION OF THE DRAWINGS

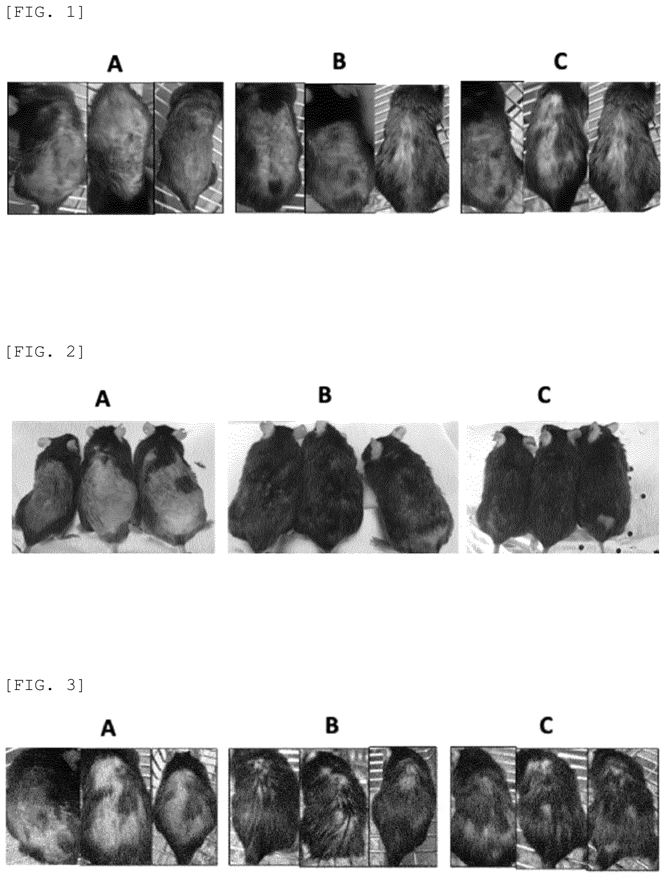

Figure 1 illustrates the hair regrowth status after application of standard lotion solution or solution containing TA-65 (100 ng/mL) or GPC (100 ng/mL) on 20-month-old C57/BL6 mice. The hair of the mice was first removed, and after confirming no hair regrowth for 4 weeks, the solutions were applied to the skin for 8 weeks. Hair regrowth was then observed. The use of 20-month-old mice is not limiting; aged mice that did not show hair regrowth within 4 weeks post-depilation were selected for the experiment.
Figure 2 shows hair regrowth after application of nanobubble-containing solutions. Specifically, (A) represents distilled water with nanobubbles, (B) represents TA-65-containing nanobubble solution, and (C) represents GPC-containing nanobubble solution. The hair of 20-month-old C57/BL6 mice was removed, and after confirming no hair regrowth for 4 weeks, the solutions were applied to the skin for 8 weeks (three times per week), and hair regrowth was observed after 8 weeks.
Figure 3 illustrates hair regrowth after application of nanoliposome solutions. Specifically, (A) represents nanoliposome solution alone, (B) represents TA-65-encapsulated nanoliposome solution, and (C) represents GPC-encapsulated nanoliposome solution. Hair was removed from 20-month-old C57/BL6 mice, and after confirming no hair regrowth for 4 weeks, the solutions were applied to the skin for 8 weeks (five times per week), followed by hair regrowth observation.

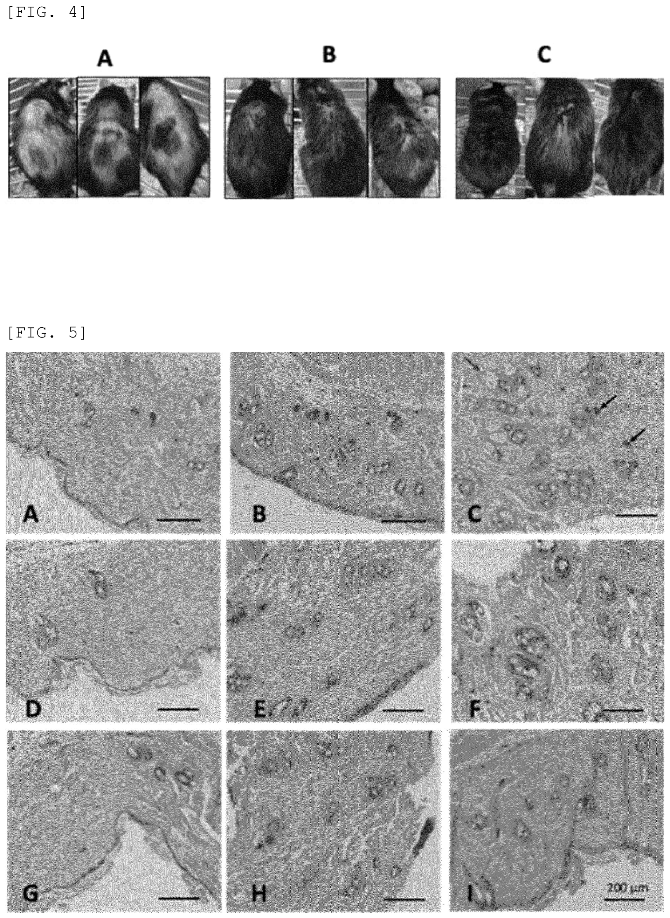

Figure 4 shows hair regrowth after application of mesoporous silica nanoparticle solutions. Specifically, (A) represents silica nanoparticle solution alone, (B) represents TA-65-loaded silica nanoparticle solution, and (C) represents GPC-loaded silica nanoparticle solution. Hair was removed from 20-month-old C57/BL6 mice, and after confirming no hair regrowth for 4 weeks, the solutions were applied to the skin for 8 weeks (five times per week), followed by hair regrowth observation.
Figure 5 shows the distribution and status of hair follicles after biopsy and H&E staining (magnification: 40×) of mouse skin used in Figures 2, 3, and 4. Representative hair follicles are indicated in Figure 5C as inactive follicles (black arrows), primary active follicles (red arrows), and secondary active follicles (yellow arrows). Specifically, the images correspond to: nanobubble solution (A), TA-65 nanobubble (B), GPC nanobubble (C), nanoliposome (D), TA-65 nanoliposome (E), GPC nanoliposome (F), silica nanoparticle (G), TA-65 silica nanoparticle (H), and GPC silica nanoparticle (I).

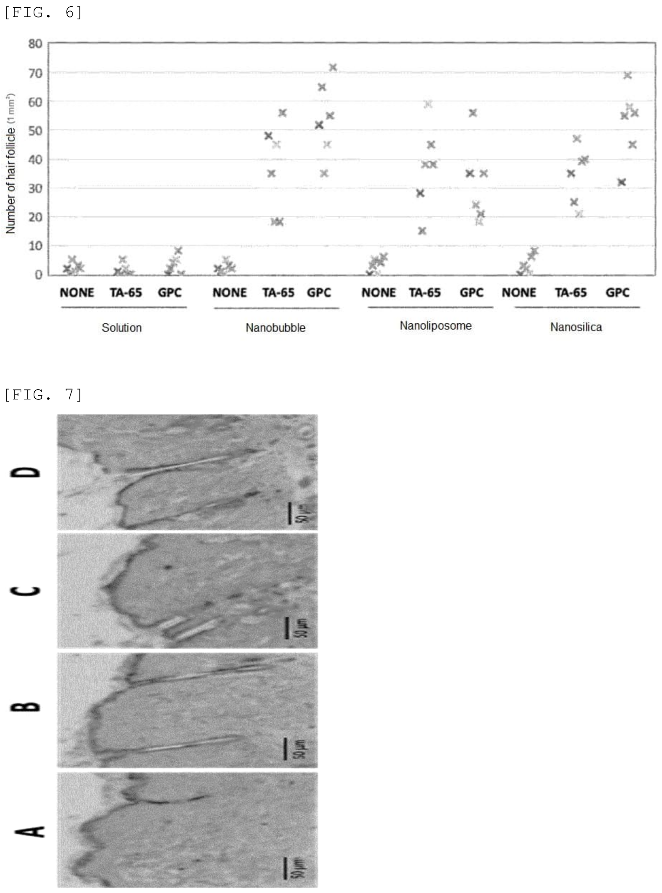

Figure 6 illustrates the increase in active hair follicles induced by telomerase-activating substances. The distribution shows the number of active follicles (primary + secondary follicles) per 1 mm², excluding inactive follicles, in H&E-stained skin tissue.
Figure 7 shows the hair follicle depth after biopsy of mouse skin treated with GPC-containing nanobubble, nanoliposome, or silica nanoparticle solutions, embedding in paraffin, sectioning laterally, and staining with H&E, as performed in Figures 2, 3, and 4.

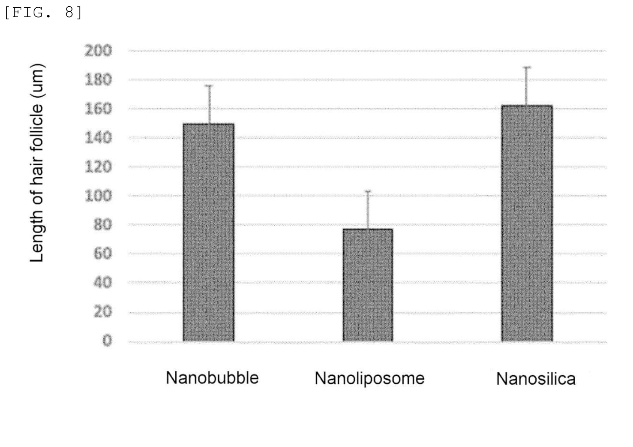

Figure 8 shows the hair follicle depth induced by GPC. Hair follicle depth was measured per 20 mm². GPC-containing nanobubble and silica nanoparticle solutions showed similar follicle depths, whereas nanoliposome-treated skin exhibited approximately 50% of that depth. In contrast, active hair follicles were scarcely observed with standard lotion solution, preventing statistical analysis.

### BEST MODE FOR CARRYING OUT THE INVENTION

In order to specifically describe the present disclosure, embodiments and experimental examples will be described in detail below. However, the present disclosure is not limited to these embodiments and experimental examples. The embodiments according to the present disclosure may be modified in various other forms, and the scope of the present disclosure should not be construed as being limited to the embodiments described below. The embodiments of the present disclosure are provided to more fully explain the present disclosure to those of ordinary skill in the art.

### [Comparative Example 1]

TA-65 (100 ng/ml) was dissolved in standard lotion solution (99.5% ethanol, 1% carbomer solution, reverse osmosis water, 1,2-hexanediol) to prepare a solution at a concentration of 100 mg/L.

### [Comparative Example 2]

GPC (100 ng/ml) was dissolved in standard lotion solution (99.5% ethanol, 1% carbomer solution, reverse osmosis water, 1,2-hexanediol) to prepare a solution at a concentration of 100 mg/L.

### [Experimental Example 1] Hair Growth Efficacy of TA-65 and GPC Dissolved in Standard Lotion Solution

To prepare animals in which hair no longer grows due to aging hair follicles, C57/BL6 mice were shaved at approximately 20 months of age (not limited to 20 months). Mice that did not exhibit hair growth for 4 weeks after shaving were selected, indicating that their hair follicles had aged and entered the full telogen phase (a state in which hair growth has ceased).

The solutions of Comparative Examples 1 and 2 were applied to the shaved skin of the selected aged mice five times per week for 8 weeks. Hair growth was then observed from week 8 to week 16. Referring to Figure 1, (A) represents skin treated with the standard lotion solution, (B) represents skin treated with the solution of Comparative Example 1, and (C) represents skin treated with the solution of Comparative Example 2. TA-65 and GPC dissolved in the standard lotion solution did not induce hair growth in aged mice.

The hair growth efficacy of TA-65 and GPC delivered via nanobubbles was confirmed in Example 1 below.

### [Example 1]

### - Preparation of a TA-65-Encapsulated Nanobubble Solution

TA-65 was dissolved in triple-distilled water at a concentration of 100 mg/L. The resulting solution was then introduced into a nanobubble housing, and nanobubbles encapsulating TA-65 were generated by applying approximately 200 W of electricity for 20 minutes, thereby preparing a TA-65-encapsulated nanobubble solution.

### [Example 2]

### - Preparation of a GPC-Encapsulated Nanobubble Solution

GPC was dissolved in triple-distilled water at a concentration of 100 mg/L. The resulting solution was then introduced into a nanobubble housing, and nanobubbles encapsulating GPC were generated by applying approximately 200 W of electricity for 20 minutes, thereby preparing a GPC-encapsulated nanobubble solution.

As a control, a nanobubble solution was prepared using triple-distilled water that did not contain either TA-65 or GPC.

### [Experimental Example 2]

### 1. Experiment on the Nanobubble Solution

As shown in Figure 2(A), mice treated with the nanobubble solution prepared from triple-distilled water did not develop any notable lesions on the skin even after 16 weeks. Furthermore, the nanobubble solution was applied five times per week for 8 weeks to the shaved skin of aged mice that had not exhibited hair growth for 4 weeks. Hair growth was then observed from week 8 onward, but no regrowth was observed even after a total of 16 weeks.

To examine the condition of hair follicles in the skin, a 4 mm-thick dorsal skin biopsy was collected from C57/BL6 mice. The skin biopsy was fixed in 10% formalin for 16 hours, and then embedded in paraffin. Horizontal sections of the tissue were prepared using a microtome, deparaffinized with xylene, and stained with Hematoxylin-Eosin (H&E) to observe the morphology and number of hair follicles, including degenerated follicles, primary follicles, and secondary follicles. In addition, vertical sections were prepared simultaneously to observe the depth (length) of the hair follicles. No abnormal findings were observed in the H&E staining; however, little increase in hair follicles was detected, and degenerated follicles were observed (Figure 5(A)).

### 2. Experiments on the Solutions of Examples 1 and 2

Following 8 weeks of application of the nanobubble solution of Example 1 containing TA-65 and the nanobubble solution of Example 2 containing GPC, hair growth was observed in almost the entire shaved skin area after 16 weeks (Figure 2(B, C)).

Skin biopsies were then collected from these mice to examine the hair follicles (Figure 5(B, C)). Compared to Figure 5(A), the number of hair follicles was significantly increased. Furthermore, compared to the follicles shown in Figure 5(B), the follicles in Figure 5(C) appeared larger and more densely arranged.

### [Example 3]

### - Preparation of a TA-65-Encapsulated Nanoliposome Solution

The composition of the cationic liposomes used in the present invention is as follows: dimyristoylphosphatidylcholine, N-(2,3-dioleoyloxy-1-propyl)trimethylammonium methyl sulfate, and cholesterol (50:10:40 mol%).

### - Method for Preparing Liposomes

First, the lipids were dissolved in a chloroform/methanol (3:1) solution and then dried under vacuum using a rotary evaporator.

A drug solution was prepared by dissolving TA-65 at a concentration of 100 mM in a solution of 10 mM N-[2-hydroxyethyl]piperazine-N'-[2-ethanesulfonic acid] + 150 mM NaCl (pH 7.5, readjusted with sodium hydroxide after dissolving the drug).

The lipid solid was then added to 2 mL of the drug solution at 60°C to obtain a final lipid concentration of 50 mM. The resulting mixture was subjected to three cycles of freezing and thawing, and subsequently extruded through polycarbonate filters with a pore size of 400 nm to prepare the TA-65-encapsulated nanoliposome solution.

### [Example 4]

### - Preparation of a GPC-Encapsulated Nanoliposome Solution

A GPC-encapsulated nanoliposome solution was prepared using the same method as described in Experimental Example 3 for the preparation of the TA-65-encapsulated nanoliposome solution, except that GPC was used instead of TA-65.

As a control, a nanoliposome solution not containing either TA-65 or GPC was prepared.

To evaluate the hair growth efficacy of TA-65 or GPC encapsulated in the nanoliposomes, the experiments described in Experimental Example 3 were conducted.

### [Experimental Example 3]

### 1. Experiment on the Nanoliposome-Containing Solution

The solution containing nanoliposomes did not induce any notable lesions on the skin even after 16 weeks (Figure 3(A)), and no abnormal findings were observed in H&E staining. However, few hair follicles were detected, and degenerated follicles were observed (Figure 5(D)).

In addition, mice that did not exhibit hair growth for 4 weeks after shaving were selected, and no hair regrowth was observed even 16 weeks thereafter (Figure 3(A)).

2. The nanoliposome solutions of Examples 3 and 4, containing TA-65 or GPC, were applied five times per week for 8 weeks to the shaved skin of aged mice that had not exhibited hair growth for 4 weeks. Hair growth was then observed from week 8 onward.

However, the nanoliposome solutions of Examples 3 and 4, containing TA-65 or GPC at a concentration of 60 mg/L, induced hair growth in almost the entire shaved skin area after 8 weeks of application and a total of 16 weeks (Figure 3(B, C)). Skin biopsies were then collected from these mice to examine the hair follicles (Figure 5(E, F)). Compared to Figure 5(D), the number of hair follicles was significantly increased. Furthermore, compared to the follicles shown in Figure 5(E), those in Figure 5(F) were larger and more densely arranged.

### [Example 5]

A TA-65-encapsulated silica nanoparticle solution was prepared by reacting TA-65 with the above-described MCM-41 under constant stirring for 48 hours (total concentration: 300 mg/L, TA-65 60 mg/L + MCM-41 240 mg/L).

### [Example 6]

A GPC-encapsulated silica nanoparticle solution was prepared by reacting GPC with the above-described MCM-41 under constant stirring for 48 hours (total concentration: 300 mg/L, GPC 60 mg/L + MCM-41 240 mg/L).

As a control, a silica nanoparticle solution not containing either TA-65 or GPC was prepared.

To evaluate the hair growth efficacy of TA-65 or GPC encapsulated in the silica nanoparticles, the experiments described in Experimental Example 4 were conducted.

### [Experimental Example 4]

1. Mice treated with the solution containing mesoporous silica nanoparticles did not develop any notable lesions on the skin even after 16 weeks (Figure 4(A)), and no abnormal findings were observed in H&E staining. However, few hair follicles were detected, and degenerated follicles were observed (Figure 5(G)). In addition, mice that did not exhibit hair growth for 4 weeks after shaving were selected, and no hair regrowth was observed even 16 weeks thereafter (Figure 4(A)).
2. However, the solutions of Examples 5 and 6, containing TA-65 or GPC at a concentration of 60 mg/L, induced hair growth in almost the entire shaved skin area after 8 weeks of application and a total of 16 weeks (Figure 4(B, C)). Skin biopsies were then collected from these mice to examine the hair follicles (Figure 5(H, I)). Compared to Figure 5(G), the number of hair follicles was significantly increased. Furthermore, compared to the follicles shown in Figure 5(H), those in Figure 5(I) were larger and more densely arranged.

Meanwhile, Figure 6 illustrates the increase in active hair follicles induced by telomerase-activating substances. The figure shows the distribution of active hair follicles (primary + secondary follicles) per square millimeter of H&E-stained skin tissue, excluding inactive follicles. In Figure 5(C), representative follicles are indicated by arrows: inactive follicles with black arrows, primary follicles with blue arrows, and secondary follicles with yellow arrows. In aged animal skin treated with telomerase-activating substances (TA-65 and GPC) dissolved in standard lotion, active follicles were scarcely observed.

In contrast, when telomerase-activating substances bound to or encapsulated in nanobubbles, nanoliposomes, or mesoporous silica nanoparticles were applied, a clear increase in the number of active hair follicles in the animal skin was observed.

Figure 7 shows H&E-stained sections of skin from mice treated with GPC-containing nanobubble solution (B), nanoliposome solution (C), or nanosilica solution (D), prepared by performing a skin biopsy, embedding the tissue in paraffin, and cutting it laterally to observe the depth of hair follicles, as similarly done in Figures 2, 3, and 4. Figure 8 shows the depth of hair follicles induced by the telomerase-activating substance GPC, measured per 20 square millimeters of skin. As shown in Figures 7 and 8, the depth of hair follicles in mice treated with GPC-containing nanobubble solution (B), nanoliposome solution (C), or nanosilica solution (D) was greater than that in mice treated with standard lotion (A). The GPC-containing nanoliposome solution (C) exhibited approximately 50% of the follicle depth observed with the GPC-containing nanobubble solution (B) and nanosilica solution (D), and active follicles were scarcely observed in the standard lotion group, making statistical analysis infeasible.

In the above-described examples, the concentrations of TA-65 and GPC used in the nanobubble, nanoliposome, and nanosilica solutions were in the range of 60-100 mg/L. However, the invention is not limited thereto, and preferably, concentrations in the range of 0.2 mg/L to 2 g/L may be used.
he invention described above is merely exemplary, and it will be apparent to those skilled in the art that various modifications and equivalent embodiments are possible therefrom. Accordingly, the invention is not intended to be limited to the forms specifically described in the foregoing detailed description. Therefore, the true technical scope of the invention should be determined by the technical spirit of the appended claims. Furthermore, the invention should be understood to encompass all modifications, equivalents, and alternatives within the scope and spirit of the invention as defined by the appended claims.

### Industrial Applicability

The pharmaceutical composition for drug delivery comprising a telomerase activator and nanoparticles, and the composition containing the same for the prevention, improvement, or treatment of hair loss, are industrially applicable.

## Claims

1. A composition for the prevention, improvement, or treatment of hair loss, comprising:
a telomerase activator; and
nanoparticles.

2. The composition according to claim 1, wherein the telomerase activator is at least one selected from the group consisting of cycloastragenol, Guanidinopentyl phenylisoxazolecarboxamide, GRN510, AGS-500, genistein, Centella asiatica extract, and maslinic acid.

3. The composition according to claim 1, wherein the nanoparticles are at least one selected from the group consisting of nanobubbles, nanoliposomes, and nanosilica, and the telomerase activator is in a conjugated structure encapsulated or entrapped within the nanoparticles.
